# EUROPEAN PATENT SPECIFICATION

(11) **EP 2 066 353 B1**
(45) Date of publication and mention of the grant of the patent: **02.01.2013**
(21) Application number: 07804404.7
(22) Date of filing: 27.09.2007
(51) Int. Cl.: C07K 16/22, A61K 31/517, A61K 39/395, A61K 31/505

(54) **COMBINATION OF ZD6474 AND BEVACIZUMAB FOR CANCER THERAPY**
KOMBINATION VON ZD6474 UND BEVACIZUMAB FÜR DIE KREBSTHERAPIE
ASSOCIATION DE ZD6474 ET DE BEVACIZUMAB POUR TRAITER LE CANCER

(30) Priority: 29.09.2006 US 827483 P
(43) Date of publication of application: 10.06.2009
(73) Proprietor: AstraZeneca AB, 151 85 Södertälje (SE)
(72) Inventor: RYAN, Anderson, Joseph, Macclesfield, Cheshire SK10 4TG (GB)
(86) International application number: PCT/GB2007/003667
(87) International publication number: WO 2008/037996

(56) References cited:
- WO-A-2006/048633
- MORGENSZTERN DANIEL ET AL: "Clinical trials of antiangiogenic therapy in non-small cell lung cancer: focus on bevacizumab and ZD6474." EXPERT REVIEW OF ANTICANCER THERAPY APR 2006, vol. 6, no. 4, April 2006 (2006-04), pages 545-551, XP009094867 ISSN: 1744-8328
- BOZEC ALEXANDRE ET AL: "Epidermal growth factor receptor/angiogenesis dual targeting: preclinical experience." CURRENT OPINION IN ONCOLOGY JUL 2006, vol. 18, no. 4, July 2006 (2006-07), pages 330-334, XP009095083 ISSN: 1040-8746
- BAKA S ET AL: "A review of the latest clinical compounds to inhibit VEGF in pathological angiogenesis" EXPERT OPINION ON THERAPEUTIC TARGETS, ASHLEY PUBLICATIONS, LONDON, GB, vol. 10, no. 6, 2006, pages 867-876, XP008080403 ISSN: 1472-8222
- CASCONE TINA ET AL: "Combined targeted therapies in non-small cell lung cancer: a winner strategy?" CURRENT OPINION IN ONCOLOGY MAR 2007, vol. 19, no. 2, March 2007 (2007-03), pages 98-102, XP009095070 ISSN: 1040-8746

## Description

The present invention relates to the production of an antiangiogenic and/or vascular permeability reducing effect in a warm-blooded animal such as a human, which is optionally being treated with ionising radiation, particularly to the treatment of a cancer, particularly a cancer involving a solid tumour, by the administration of ZD6474 in combination with bevacizumab; to a pharmaceutical composition comprising ZD6474 and bevacizumab; to a combination product comprising ZD6474 and bevacizumab for use in the treatment of a human or animal body by therapy; to a kit comprising ZD6474 and bevacizumab; to the use of ZD6474 and bevacizumab in the manufacture of a medicament for use in the production of an antiangiogenic and/or vascular permeability reducing effect in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with in vitro endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36:139-155) and vascular permeability (Connolly et al, 1989, J. Biol. Chem. 264: 20017-20024). Antagonism of VPGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1 (also referred to as VEGFR-1), the kinase insert domain-containing receptor, KDR (also referred to as VEGFR-2 or Flk-1), and another fins-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem., 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Applications Publication Nos. WO 98/13354 and WO 01/32651. In WO 98/13354 and WO 01/32651 compounds are described which possess activity against VEGF receptor tyrosine kinase (VEGF RTK) whilst possessing some activity against epidermal growth factor (EGF) receptor tyrosine kinase (EGF RTK). ZD6474 is 4-(4-bromo-2-fluoroanilino)-6-methoxy-7-(1-methylpiperidin-4-ylmethoxy)quinazoline:

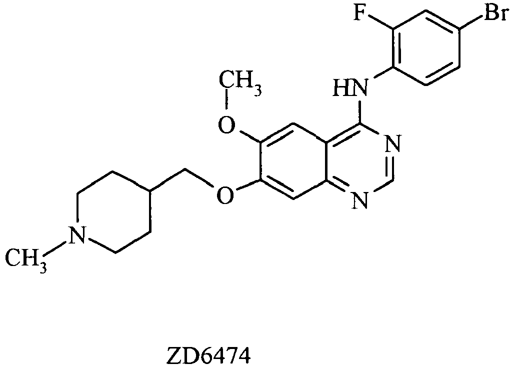

ZD6474 is also known as vandetanib and as ZACTIMA^{™} (AstraZeneca).

ZD6474 falls within the broad general disclosure of WO 98/13354 and is exemplified in WO 01/32651. ZD6474 is a potent inhibitor of VEGF RTK and also has some activity against EGF RTK. ZD6474 has been shown to elicit broad-spectrum anti-tumour activity in a range of models following once-daily oral administration (Wedge SR, Ogilvie DJ, Dukes M, et al. ZD6474 inhibits vascular endothelial growth factor signaling, angiogenesis, and tumour growth following oral administration. Cancer Res 2002;62:4645-4655).

In Morgensztem Daniel et al: Expert Review of Anticancer Therapy Apr 2006, vol. 6, no. 4, April 2006, pages 545-551, the use of ZD6474 in combination wth paclitaxel and carboplatin in patients with non small cell lung cancer (NSCLC) is described, also the use of ZD6474 and docetaxel in NSCLC. In the same paper it describes the use of bevacizumab in combination with cytotoxic chemotherapy for the treatment of NSCLC.

In Bozec Alexandre et al: Current Opinion in Oncology Jul 2006, vol. 18, no. 4, July 2006, pages 330-334, is described the use of bevacizumab and erlotinib, an inhibitor of EGF RTK in NSCLC.

In WO 2006/048633 combinations of ZD6474 and androgen ablation are described.

Baka S et al: Expert Opinion on Therapeutic Targets, Ashley Publications, London, GB, vol. 10, no. 6, 2006, pages 867-876, describes several VEGF inhibitors including ZD6474 and bevacizumab as does Cascone Tina et al: Current Opinion in Oncology Mar 2007, vol. 19, no. 2, March 2007, pages 98-102 which focuses on possible treatments for NSCLC.

In WO 98/13354 and WO 01/32651 it is stated that compounds of their inventions: "may be applied as a sole therapy or may involve, in addition to a compound of the invention, one or more other substances and/or treatments. Such conjoint treatment may be achieved by way of the simultaneous, sequential or separate administration of the individual components of the treatment."

WO 98/13354 and WO 01/32651 then go on to describe examples of such conjoint treatment including surgery, radiotherapy and various types of chemotherapeutic agent.

Nowhere in WO 98/13354 and WO 01/32651 do they suggest the combination of a compound of the invention and bevacizumab for the treatment of any disease state (including cancer.

Nowhere in WO 98/13354 and WO 01/32651 is the specific combination of ZD6474 and bevacizumab suggested.

Nowhere in WHO 98/13354 and WO 01/32651 does it state that use of any compound of the invention therein with other treatments will produce surprisingly beneficial effects.

Bevacizumab is a recombinant humanized monoclonal antibody to the vascular endothelial growth factor ligand VEGF-A. Bevacizumab binds to VEGF-A and thereby inhibits the binding of VEGF-A to its receptors Flt-1 and KDR. Bevacizumab is produced by DNA technology in Chinese Hamster ovary cells.

Bevacizumab is also known as AVASTIN^{™} (Genentech Inc; Roche Pharmaceuticals).

Anti-cancer effects of a treatment of the present invention include, but are not limited to, anti-tumour effects, the response rate, the time to disease progression and the survival rate. Anti-tumour effects of a treatment of the present invention include but are not limited to, inhibition of tumour growth, tumour growth delay, regression of tumour, shrinkage of tumour, increased time to regrowth of tumour on cessation of treatment, slowing of disease progression. It is expected that when a treatment of the present invention is administered to a warm-blooded animal such as a human, in need of treatment for cancer, said method of treatment will produce an effect, as measured by, for example, one or more of: the extent of the anti-tumour effect, the response rate, the time to disease progression and the survival rate. Anti-cancer effects include prophylactic treatment as well as treatment of existing disease.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of bevacizumab.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of bevacizumab.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab, wherein the cancer is a cancer of the colon (including the rectum), pancreas, liver, oesophagus, stomach, kidney, bladder, thyroid, head and neck, brain (for example glioma), cervix, vulva, ovary, breast, prostate, lungs or skin or is one of the haematological malignancies.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab, wherein the cancer is a cancer of the colon (including the rectum), kidney, brain (for example glioma), breast or lung.

A method for the treatment of colorectal cancer in a warm-blooded animal such as a human, may comprise administering to said animal an effective amount of ZD6474 or a pharmaceutically acceptable salt thereof, before, after or simultaneously with an effective amount of bevacizumab.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of bevacizumab.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier and wherein the cancer is a cancer of the colon (including the rectum), pancreas, liver, oesophagus, stomach, kidney, bladder, thyroid, head and neck, brain, cervix, vulva, ovary, breast, prostate, lungs or skin or is one of the haematological malignancies.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier and wherein the cancer is a cancer of the colon (including the rectum), kidney, brain (for example glioma), breast, or lung.

A method for the treatment of colorectal cancer in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of bevacizumab; wherein ZD6474 and bevacizumab may each optionally be administered together with a pharmaceutically acceptable excipient or carrier.

According to an aspect of the invention there is provided a pharmaceutical composition which comprises ZD6474 or a pharmaceutically acceptable salt thereof, and bevacizumab in association with a pharmaceutically acceptable excipient or carrier.

According to a further aspect of the present invention there is provided a combination product comprising ZD6474 or a pharmaceutically acceptable salt thereof and bevacizumab, for use in a method of treatment of a human or animal body by therapy.

According to a further aspect of the present invention there is provided a kit comprising ZD6474 or a pharmaceutically acceptable salt thereof, and bevacizumab.

According to a further aspect of the present invention there is provided a kit comprising:
a) ZD6474 or a pharmaceutically acceptable salt thereof in a first unit dosage form;
b) bevacizumab in a second unit dosage form; and
c) container means for containing said first and second dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) bevacizumab together with a pharmaceutically acceptable excipient or carrier, in a second unit dosage form; and
c) container means for containing said first and second dosage forms.

There may be provided the use of ZD6474 or a pharmaceutically acceptable salt thereof and bevacizumab in the manufacture of a medicament for use in the production of an antiangiogenic and/or vascular permeability reducing effect in a warm-blooded animal such as a human.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human.

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human wherein the cancer is a cancer of the colon (including the rectum), pancreas, liver, oesophagus, stomach, kidney, bladder, thyroid, head and neck, brain, cervix, vulva, ovary, breast, prostate, lungs or skin or is one of the haematological malignancies.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human wherein the cancer is a cancer of the colon (including the rectum), kidney, brain (for example glioma), breast or lung.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human wherein the cancer is colorectal cancer.

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human wherein the tumour is a tumour of the colon or rectum.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human wherein the cancer is non-small cell lung cancer (NSCLC).

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human wherein the tumour is a non-small cell tumour of the lung.

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human wherein the tumour is a non-small cell tumour of the lung, or a tumour of the colon or rectum or a tumour of the brain (for example glioma), kidney or breast.

According to a further aspect of the present invention there is provided a combination treatment comprising the administration of an effective amount of ZD6474 or a pharmaceutically acceptable salt thereof, optionally together with a pharmaceutically acceptable excipient or carrier, and the simultaneous, sequential or separate administration of an effective amount of bevacizumab; wherein bevacizumab may optionally be administered together with a pharmaceutically acceptable excipient or carrier; to a warm-blooded animal such as a human in need of such therapeutic treatment.
Such therapeutic treatment includes an antiangiogenic and/or vascular permeability effect, an anti-cancer effect and an anti-tumour effect.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent in addition to a combination treatment of the invention. Surgery may comprise the step of partial or complete tumour resection, prior to, during or after the administration of the combination treatment with ZD6474 described herein.

Other chemotherapeutic agents for optional use with a combination treatment of the present invention include those described in WO 01/32651. Such chemotherapy may cover five main categories of therapeutic agent:
(i) other antiangiogenic agents including vascular targeting agents;
(ii) cytostatic agents including growth factor inhibitors such as VEGF RTK inhibitors like sorafenib and sunitinib, EGF RTK inhibitors such as cetuximab, panitumumab, gefitinib and erlotinib, and c-kit inhibitors such as imatinib;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.
Particular examples of chemotherapeutic agents for use with a combination treatment of the present invention are raltitrexed, pemetrexed, etoposide, vinorelbine, paclitaxel, docetaxel, cisplatin, oxaliplatin, carboplatin, gemcitabine, topotecan, irinotecan (CPT-11), 5-fluorouracil (5-FU, (including capecitabine)), doxorubicin, cyclophosphamide, temozolomide, hydroxyurea, sorafenib, sunitinib, cetuximab, panitumumab, gefitinib, erlotinib, and imatinib. Such combinations are expected to be particularly useful for the treatment of cancer of the colon (including the rectum), pancreas, liver, oesophagus, stomach, kidney, bladder, thyroid, head and neck, brain, cervix, vulva, ovary, breast, prostate, lungs and skin and including haematological malignancies. Such combinations are expected to be more particularly useful for the treatment of colorectal cancer, non-small cell lung cancer (NSCLC), renal cancer, brain tumours (for example glioma) and breast cancer. Such combinations are expected to be more particularly useful for the treatment of colorectal cancer, non-small cell lung cancer (NSCLC) and breast cancer.

The administration of a triple combination of ZD6474, bevacizumab and an EGF RTK inhibitor such as cetuximab, panitumumab, gefitinib or erlotinib may produce effects, such as anti-tumour effects, greater than those achieved with any of ZD6474, bevacizumab and an EGF RTK inhibitor used alone, greater than those achieved with the combination of ZD6474 and bevacizumab, greater than those achieved with the combination of ZD6474 and an EGF RTK inhibitor, greater than those achieved with the combination of bevacizumab and an EGF RTK inhibitor.

There may be provided the use of ZD6474 or a pharmaceutically acceptable salt thereof and bevacizumab and an inhibitor of epidermal growth factor receptor tyrosine kinase in the manufacture of a medicament for use in the production of an antiangiogenic and/or vascular permeability reducing effect in a warm-blooded animal such as a human.

According to a further aspect of the present invention there is provided the use of ZD6474 or a pharmaceutically acceptable salt thereof and bevacizumab and an inhibitor of epidermal growth factor receptor tyrosine kinase in the manufacture of a medicament for use in the production of an anti-cancer effect in a warm-blooded animal such as a human.

According to a further aspect of the present invention there is provided the use of ZD6474 or a pharmaceutically acceptable salt thereof and bevacizumab and an inhibitor of epidermal growth factor receptor tyrosine kinase in the manufacture of a medicament for use in the production of an anti-tumour effect in a warm-blooded animal such as a human.

According to a further aspect of the present invention there is provided the use of ZD6474 or a pharmaceutically acceptable salt thereof and bevacizumab and an inhibitor of epidermal growth factor receptor tyrosine kinase in the manufacture of a medicament for use in the production of an anti-cancer effect in a warm-blooded animal such as a human wherein the cancer is a cancer of the colon (including the rectum), kidney, brain (for example glioma), breast or lung.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of an EGF RTK inhibitor.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of an EGF RTK inhibitor.

An method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of an EGF RTK inhibitor.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of an EGF RTK inhibitor, wherein the cancer is a cancer of the colon (including the rectum), kidney, brain (for example glioma), breast or lung.

According to a further aspect of the present invention there is provided a combination treatment comprising the administration of an effective amount of ZD6474 or a pharmaceutically acceptable salt thereof, optionally together with a pharmaceutically acceptable excipient or carrier, and the simultaneous, sequential or separate administration of an effective amount of bevacizumab, wherein bevacizumab may optionally be administered together with a pharmaceutically acceptable excipient or carrier, and the simultaneous, sequential or separate administration of an effective amount of an EGF RTK inhibitor, optionally together with a pharmaceutically acceptable excipient or carrier, to a warm-blooded animal such as a human in need of such therapeutic treatment.
Particular EGF RTK inhibitors include cetuximab, panitumumab, gefitinib and erlotinib.

The administration of a triple combination of ZD6474, bevacizumab and ionising radiation may produce effects, such as anti-tumour effects, greater than those achieved with any of ZD6474, bevacizumab and ionising radiation used alone, greater than those achieved with the combination of ZD6474 and bevacizumab, greater than those achieved with the combination of ZD6474 and ionising radiation, greater than those achieved with the combination of bevacizumab and ionizing radiation.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an affective amount of ionising radiation.

A method for the treatment of a cancer in a warm-blooded animal such as a human may comprise administering to said animal an effective amount of ZD6474 or a pharmaceutically acceptable salt thereof, before, after or simultaneously with an effective amount of bevacizumab and before, after or simultaneously with an effective amount of ionising radiation.

A method for the treatment of a cancer involving a solid tumour in a warm-blooded animal such as a human, may comprise administering to said animal an effective amount of ZD6474 or a pharmaceutically acceptable salt thereof, before, after or simultaneously with an affective amount of bevacizumab and before, after or simultaneously with an effective amount of ionising radiation.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein the cancer is a cancer of the colon (including the rectum), pancreas, liver, oesophagus, stomach, kidney, bladder, thyroid, head and neck, brain, cervix, vulva, ovary, breast, prostate, lungs or skin or is one of the haematological malignancies.

A method for the treatment of a cancer in a warm-blooded animal such as a human may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein the cancer is a cancer of the colon (including the rectum), kidney, brain (for example glioma), breast or lung.

A method for the treatment of colorectal cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier. A

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier, and wherein the cancer is a cancer of the colon (including the rectum), pancreas, liver, oesophagus, stomach, kidney, bladder, thyroid, head and neck, brain, cervix, vulva, ovary, breast, prostate, lungs or skin or is one of the haematological malignancies.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier, and wherein the cancer is a cancer of the colon (including the rectum), kidney, brain (for example glioma), breast or lung.

A method for the treatment of colorectal cancer in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of bevacizumab and before, after or simultaneously with an effective amount of ionising radiation, wherein ZD6474 and bevacizumab may each optionally be administered together with a pharmaceutically acceptable excipient or carrier.

There may be provided the use of ZD6474 or a pharmaceutically acceptable salt thereof and bevacizumab in the manufacture of a medicament for use in the production of an antiangiogenic and/or vascular permeability reducing effect in a warm-blooded animal such as a human which is being treated with ionising radiation.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human which is being treated with ionising radiation.

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human which is being treated with ionising radiation.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human which is being treated with ionising radiation, wherein the cancer is a cancer of the colon (including the rectum), pancreas, liver, oesophagus, stomach, kidney, bladder, thyroid, head and neck, brain, cervix, vulva, ovary, breast, prostate, lungs or skin or is one of the haematological malignancies.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human which is being treated with ionising radiation, wherein the cancer is a cancer of the colon (including the rectum), kidney, brain (for example glioma), breast, or lung.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human which is being treated with ionising radiation wherein the cancer is colorectal cancer.

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human which is being treated with ionising radiation wherein the tumour is a tumour of the colon or rectum.

According to a further aspect of the present invention there is provided the use of ZD6474 or a pharmaceutically acceptable salt thereof and bevacizumab in the manufacture of a medicament for use in the production of an anti-cancer effect in a warm-blooded animal such as a human which is being treated with ionising radiation wherein the cancer is non-small cell lung cancer (NSCLC).

According to a further aspect of the present invention there is provided the use of ZD6474 or a pharmaceutically acceptable salt thereof and bevacizumab in the manufacture of a medicament for use in the production of an anti-tumour effect in a warm-blooded animal such as a human which is being treated with ionising radiation wherein the tumour is a non-small cell tumour of the lung.

According to a further aspect of the present invention there is provided the use of Z6474 or a pharmaceutically acceptable salt thereof and bevacizumab in the manufacture of a medicament for use in the production of an anti-tumour effect in a warm-blooded animal such as a human which is being treated with ionising radiation wherein the tumour is a tumour of the colon or rectum, kidney, brain (for example glioma), breast or lung.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of bevacizumab, optionally together with a pharmaceutically acceptable excipient or carrier and the administration of an effective amount of ionising radiation, to a warm-blooded animal such as a human in need of such therapeutic treatment wherein the ZD6474, bevacizumab and ionising radiation may be administered simultaneously, sequentially or separately and in any order.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab, and before, after or simultaneously with an effective amount of an EGF RTK inhibitor and before, after or simultaneously with an effective amount of ionising radiation, wherein the cancer is a cancer of the colon (including the rectum), pancreas, liver, oesophagus, stomach, kidney, bladder, thyroid, head and neck, brain (for example glioma), cervix, vulva, ovary, breast, prostate, lungs or skin or is one of the haematological malignancies.

A method for the treatment of a cancer in a warm-blooded animal such as a human, may comprise administering to said animal an effective amount of ZD6474 or a pharmaceutically acceptable salt thereof, before, after or simultaneously with an effective amount of bevacizumab, and before, after or simultaneously with an effective amount of an EGF RTK inhibitor and before, after or simultaneously with an effective amount of ionising radiation, wherein the cancer is a cancer of the colon (including the rectum), kidney brain (for example glioma), breast or lung.

According to a further aspect of the present invention there is provided the use of ZD6474 or a pharmaceutically acceptable salt thereof and bevacizumab and an EGF RTK inhibitor in the manufacture of a medicament for use in the production of an anti-cancer effect in a warm-blooded animal such as a human which is being treated with ionising radiation, wherein the cancer is a cancer of the colon (including the rectum), pancreas, liver, oesophagus, stomach, kidney, bladder, thyroid, head and neck, brain, cervix, vulva, ovary, breast, prostate, lungs or skin or is one of the haematological malignancies.

According to a further aspect of the present invention there is provided the use of ZD6474 or a pharmaceutically acceptable salt thereof and bevacizumab and an EGF RTK inhibitor in the manufacture of a medicament for use in the production of an anti-cancer effect in a warm-blooded animal such as a human which is being treated with ionising radiation, wherein the cancer is a cancer of the colon (including the rectum), kidney, brain (for example glioma), breast, or lung.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of bevacizumab, optionally together with a pharmaceutically acceptable excipient or carrier, and the administration of an effective amount of an EGF RTK inhibitor, optionally together with a pharmaceutically acceptable excipient or carrier, and the administration of an effective amount of ionising radiation, to a warm-blooded animal such as a human in need of such therapeutic treatment wherein the ZD6474, bevacizumab, EGF RTK inhibitor and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising ZD6474 and bevacizumab. For example said ionising radiation may be given to said warm-blooded animal such as a human within the period of a week before to a week after the administration of a medicament or combination treatment comprising ZD6474 and bevacizumab. This means that ZD6474, bevacizumab and ionising radiation may be administered separately or sequentially in any order, or may be administered simultaneously. The warm-blooded animal may experience the effect of each of ZD6474, bevacizumab and radiation simultaneously.

According to one aspect of the present invention the ionising radiation is administered before one of ZD6474 and bevacizumab or after one of ZD6474 and bevacizumab.

According to one aspect of the present invention the ionising radiation is administered before both ZD6474 and bevacizumab or after both ZD6474 and bevacizumab.

According to one aspect of the present invention ZD6474 is administered to a warm-blooded animal after the animal has been treated with ionising radiation.

According to another aspect of the present invention the effect of a treatment of the present invention is expected to be at least equivalent to the addition of the effects of each of the components of said treatment used alone, that is, of each of ZD6474 and bevacizumab used alone or of each of ZD6474, bevacizumab and ionizing radiation used alone.

According to another aspect of the present invention the effect of a treatment of the present invention is expected to be greater than the addition of the effects of each of the components of said treatment used alone, that is, of each of ZD6474 and bevacizumab used alone or of each of ZD6474, bevacizumab and ionising radiation used alone.

According to another aspect of the present invention the effect of a treatment of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with ZD6474 or bevacizumab or ionising radiation alone. Further, the effect of the combination treatment is synergistic if a beneficial effect is obtained in a group of patients that does not respond (or responds poorly) to ZD6474 or bevacizumab or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of ZD6474 or bevacizumab or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments as defined herein are of interest for their antiangiogenic and/or vascular permeability effects. Angiogenesis and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, asthma, lymphoedema, endometriosis, dysfunctional uterine bleeding and ocular diseases with retinal vessel proliferation including age-related macular degeneration.

Combination treatments of the present invention are expected to be particularly useful in the prophylaxis and treatment of diseases such as cancer and Kaposi's sarcoma. Combination treatments of the present invention are expected to slow advantageously the growth of tumours in colorectal cancer, lung cancer, including malignant pleural mesothelioma, small cell lung cancer (NSCLC) and non-small cell lung cancer (NSCLC), breast cancer, renal cancer, bladder cancer, oesophageal cancer, pancreatic cancer, head and neck cancer, thyroid cancer, skin cancer including melanoma, hepatocellular carcinoma, glioma, neuroblastoma, gastric cancer, prostate cancer, cervical cancer, cancer of the vulva and ovarian cancer and also in the haematological malignances such as leukaemia, multiple myeloma and lymphoma. In particular such combination treatments of the invention are expected to slow advantageously the growth of primary and recurrent solid tumours of, for example, the colon (including the rectum), pancreas, liver, oesophagus, stomach, kidney, bladder, thyroid, head and neck, brain, cervix, ovary, breast, prostate, lungs and skin and including haematological malignancies. Combination treatments of the present invention are expected to slow advantageously the growth of tumours in colorectal cancer, breast cancer and lung cancer, including malignant pleural mesothelioma, small cell lung cancer (SCLC) and non-small cell lung cancer (NSCLC). More particularly such combination treatments of the invention are expected to inhibit any form of cancer associated with VEGF including leukaemia, multiple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), pancreas, liver, oesophagus, stomach, thyroid, head and neck, brain, kidney, bladder, cervix, vulva, ovary, breast, prostate, lungs and skin and particularly colorectal cancer and NSCLC. More especially combination treatments of the present invention are expected to slow advantageously the growth of tumours in colorectal cancer. More especially combination treatments of the present invention are expected to slow advantageously the growth of tumours in non-small cell lung cancer (NSCLC). More especially combination treatments of the present invention are expected to slow advantageously the growth of tumours in breast cancer. More especially combination treatments of the present invention are expected to slow advantageously the growth of tumours in pancreatic cancer. More especially combination treatments of the present invention are expected to slow advantageously the growth of tumours in hepatocellular carcinoma. More especially combination treatments of the present invention are expected to slow advantageously the growth of tumours in gastric cancer. More especially combination treatments of the present invention are expected to slow advantageously the growth of tumours in thyroid cancer. More especially combination treatments of the present invention are expected to slow advantageously the growth of tumours in cancer of the head and neck. More especially combination treatments of the present invention are expected to slow advantageously the growth of tumours of the brain such as gliomas and neuroblastomas. More especially combination treatments of the present invention are expected to slow advantageously the growth of tumours in renal cancer. More especially combination treatments of the present invention are expected to slow advantageously the growth of tumours in bladder cancer. More especially combination treatments of the present invention are expected to slow advantageously the growth of tumours in ovarian cancer. More especially combination treatments of the present invention are expected to slow advantageously the growth of tumours in prostate cancer. More especially combination treatments of the present invention are expected to slow advantageously the growth of tumours in skin cancer. More especially combination treatments of the present invention are expected to slow advantageously the growth of tumours in cancer of the vulva. More especially combination treatments of the present invention are expected to slow advantageously the growth of tumours in cervical cancer. More especially combination treatments of the present invention are expected to slow advantageously the growth of tumours in cancer of the oesophagus. More especially combination treatments of the present invention are expected to slow advantageously the growth of tumours in haematological malignances such as leukaemia, multiple myeloma and lymphoma.

In another aspect of the present invention ZD6474 and bevacizumab, optionally with ionising radiation, are expected to inhibit the growth of those primary and recurrent solid tumours which are associated with VEGF especially those tumours which are significantly dependent on VEGF for their growth and spread.

In another aspect of the present invention ZD6474 and bevacizumab, optionally with ionising radiation, are expected to inhibit the growth of those primary and recurrent solid tumours which are associated with both VEGF and EGF especially those tumours which are significantly dependent on VEGF and EGF for their growth and spread.

The compositions described herein may be in a form suitable for oral administration, for example as a tablet, capsule, liquid or suspension, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6474 of the combination treatment may be delivered endoscopically, intratracheally, intralesionally, percutaneously, intravenously, subcutaneously, intraperitoneally or intratumourally.
Preferably ZD6474 is administered orally.
Preferably bevacizumab is administered intravenously, preferably by intravenous infusion.

In general the compositions described herein may be prepared in a conventional manner using conventional excipients. The compositions of the present invention are advantageously presented in unit dosage form.

ZD6474 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-500mg of active ingredient. Preferably a daily dose in the range of 0.5-5mg/kg is employed.

Bevacizumab may be administered according to known clinical practice.

For example in first line treatment of patients with metastatic carcinoma of the colon or rectum bevacizumab may be given at a dose of 5mg/kg of body weight given once every 14 days as an intravenous infusion. The initial infusion should be given over 90 minutes. If the first infusion is well tolerated the second infusion may be given over 60 minutes. If the 60-minute infusion is well tolerated all subsequent infusions may be administered over 30 minutes.

For example, for the treatment of colorectal cancer bevacizumab used in combination with intravenous 5-FU-based chemotherapy, may be administered as an intravenous infusion (5mg/kg or 10mg/kg) every 14 days until disease progression.

The recommended dose of bevacizumab, when used in combination with bolus-IFL (irinotecan, 5-FU, leucovorin), is 5mg/kg.

The recommended dose of bevacizumab, when used in combination with FOLFOX4 (oxaliplatin, folinic acid and 5-FU), is 10 mg/kg.

For example in NSCLC bevacizumab may be given at a dose of 15mg/kg every three weeks.

For example in NSCLC bevacizumab may be given at a dose of 7.5mg/kg every three weeks.

For example in metastatic breast cancer the recommended dose of bevacizumab is 10mg/kg given once every 2 weeks or 15mg/kg given once every 3 weeks as an intravenous infusion.

The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of y-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1 Gy per hour over a number of days. Dosage ranges for radioisotopes vary widely, and depend on the half-life of the isotope, the strength and type of radiation emitted, and on the uptake by cells.

The size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the abovementioned doses of the components of the combination treatments in order to reduce toxicity.

The present invention relates to combinations of bevacizumab with ZD6474 or with a salt of ZD6474.

Salts of ZD6474 for use in pharmaceutical compositions will be pharmaceutically acceptable salts, but other salts may be useful in the production of ZD6474 and its pharmaceutically acceptable salts: Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

ZD6474 may be synthesised according to any of the known processes for making ZD6474. For example ZD6474 may be made according to any of the processes described in WO 01/32651; for example those described in Examples 2(a), 2(b) and 2(c) of WO 01/32651. For example ZD6474 may be made according to any of the processes described in WO 07/036713.

Bevacizumab is commercially available.
The following tests may be used to demonstrate the activity of ZD6474 in combination with bevacizumab.

### A431 human vulval epidermoid carcinoma xenograft model

Experiments were conducted on female athymic mice (Swiss *mu*/*nu* genotype, ≥6 weeks of age). A431 human tumour xenografts were established in the experimental mice by injecting 1x10⁷ cells per mouse, mixed in a ratio of 1:1 with Matrigel, subcutaneously in the dorsal flank in a volume of 100µl. Tumour volumes were assessed at least twice weekly by bilateral Vernier calliper measurements. Mice were randomised into treatment groups when the tumour volume reached 0.2-0.4cm³ (day 0, this was after 7 days in Example 1 below). Following randomisation, mice were treated with either drug vehicle (Control) or ZD6474 (25 mg/kg/day) administered orally (p.o.) once daily from day 0 until end of the study, or with bevacizumab (0.5 mg/kg, injected intraperitoneally (i.p.) twice a week from day 0 until the end of the study. An additional group of animals received a combination of ZD6474 and bevacizumab, using the same doses and schedules as used for single agent treatment.

Tumour growth inhibition from the start of treatment was assessed by comparison of the differences in tumour volume (median and geometric mean) between control and treated groups. The effects of combination treatment were assessed by comparing any effect on tumour growth in the group of animals receiving ZD6474 plus bevacizumab with tumour growth in the groups where animals received single agent therapy alone.

An analogous experiment may be used to look at the combination of ZD6474 and bevacizumab with ionising radiation.

An analogous experiment may be used to look at the combination of ZD6474 and bevacizumab and an EGF RTK.

An analogous experiment may be used to look at the combination of ZD6474 and bevacizumab and an EGF RTK with ionising radiation.

### Example 1

Following the methodology described above mice were randomised into four treatment groups 7 days after tumour cell implantation (mean tumor start size 0.21 cm³). Mice were treated with daily oral doses of ZD6474, or twice weekly (Tuesday and Friday) i.p. doses of bevacizumab, either alone (Groups II and III), or in combination (Group IV). Control animals (Group I) received drug vehicles alone. Data in Table I show tumour volumes 20 days after randomisation when control tumours reached approximately 1.0 cm³.

**Table I: Antitumour effects of bevacizumab and ZD6474 alone and in combination in the A431 human tumor xenograft model in nude mice**

| Treatment Group | A431 tumor volume (cm³) | |
|---|---|---|
| | Geometric mean | Median [Interquartile range} |
| | | |
| I. Control (drug vehicles) (n=14) | 1.048 | 1.047 [0.919, 1.132] |
| II. Bevacizumab (0.5 mg/kg i.p.) (n=9) | 0.553 | 0.528 [0.494, 0.590] |
| III. ZD6474 (25 mg/kg p.o.) (n=10) | 0.149 | 0.164 [0.107, 0.203] |
| IV. Bevacizumab + ZD6474 (n=10) | 0.114 | 0.127 [0.075, 0.166] |

The data show that the combination of ZD6474 and Avastin produces greater antitumor effects, as evidenced by lower tumour volume, than either agent alone.

## Claims

1. ZD6474 or a pharmaceutically acceptable salt thereof and bevacizumab for use in the treatment of cancer in a warm-blooded animal such as a human.

2. ZD6474 or a pharmaceutically acceptable salt thereof and bevacizumab for use in the treatment of cancer according to claim 1 wherein the warm-blooded animal such as a human is being treated with ionising radiation.

3. ZD6474 or a pharmaceutically acceptable salt thereof and bevacizumab for use in the treatment of cancer according to claim 1 or claim 2 wherein the cancer is colorectal cancer, lung cancer, including malignant pleural mesothelioma, small cell lung cancer (SCLC) and non-small cell lung cancer (NSCLC), breast cancer, renal cancer, bladder cancer, oesophageal cancer, pancreatic cancer, head and neck cancer, thyroid cancer, skin cancer including melanoma, hepatocellular carcinoma, glioma, neuroblastoma, gastric cancer, prostate cancer, cervical cancer, cancer of the vulva or ovarian cancer or one of the haematological malignances.

4. ZD6474 or a pharmaceutically acceptable salt thereof and bevacizumab for use in the treatment of cancer according to claim 1 or claim 2 wherein the cancer is non-small cell lung cancer (NSCLC) or colorectal cancer or cancer or the kidney, brain or breast.

5. ZD6474 or a pharmaceutically acceptable salt thereof and bevacizumab for use in the treatment of cancer according to claim 1 or claim 2 wherein the cancer is non-small cell lung cancer (NSCLC) or colorectal cancer.

6. A pharmaceutical composition which comprises ZD6474 or a pharmaceutically acceptable salt thereof, and bevacizumab in association with a pharmaceutically acceptable excipient or carrier.

7. A kit comprising ZD6474 or a pharmaceutically acceptable salt thereof and bevacizumab.

8. ZD6474 or a pharmaceutically acceptable salt thereof and bevacizumab for use in the treatment of cancer according to claim 1 or claim 2 wherein ZD6474 or a pharmaceutically acceptable salt thereof and bevacizumab are administered simultaneously, sequentially or separately.

9. ZD6474 or a pharmaceutically acceptable salt thereof and bevacizumab and an inhibitor of epidermal growth factor receptor tyrosine kinase for use in the treatment of cancer in a warm-blooded animal such as a human.

## Patentansprüche

1. ZD6474 oder ein pharmazeutisch annehmbares Salz davon und Bevacizumab zur Verwendung bei der Behandlung von Krebs in einem Warmblüter wie einem Menschen.

2. ZD6474 oder ein pharmazeutisch annehmbares Salz davon und Bevacizumab zur Verwendung bei der Behandlung von Krebs nach Anspruch 1, wobei der Warmblüter, z. B. ein Mensch, mit ionisierender Strahlung behandelt wird.

3. ZD6474 oder ein pharmazeutisch annehmbares Salz davon und Bevacizumab zur Verwendung bei der Behandlung von Krebs nach Anspruch 1 oder 2, wobei es sich bei dem Krebs um Kolorektalkrebs, Lungenkrebs einschließlich malignem pleuralem Mesotheliom, kleinzelligem Lungenkrebs (Small Cell Lung Cancer , SCLC) und nichtkleinzelligem Lungenkrebs (Non-Small Cell Lung Cancer, NSCLC), Brustkrebs, Nierenkrebs, Blasenkrebs, Speiseröhrenkrebs, Bauchspeicheldrüsenkrebs, Kopf- und Halskrebs, Schildrüsenkrebs, Hautkrebs einschließlich Melanom, hepatozellulärem Karzinom, Gliom, Neuroblastom, Magenkrebs, Prostatakrebs, Gebährmutterhalskrebs, Krebs der Vulva oder Eierstockkrebs oder eine der hämatologischen Malignitäten handelt.

4. ZD6474 oder ein pharmazeutisch annehmbares Salz davon und Bevacizumab zur Verwendung bei der Behandlung von Krebs nach Anspruch 1 oder 2, wobei es sich bei dem Krebs um nichtkleinzelligen Lungenkrebs (NSCLC) oder Kolorektalkrebs oder Nierenkrebs, Hirnkrebs oder Brustkrebs handelt.

5. ZD6474 oder ein pharmazeutisch annehmbares Salz davon und Bevacizumab zur Verwendung bei der Behandlung von Krebs nach Anspruch 1 oder 2, wobei es sich bei dem Krebs um nichtkleinzelligen Lungenkrebs (NSCLC) oder Kolorektalkrebs handelt.

6. Pharmazeutische Zusammensetzung, welche ZD6474 oder ein pharmazeutisch annehmbares Salz davon und Bevacizumab zusammen mit einem pharmazeutisch annehmbaren Exzipienten oder Träger enthält.

7. Kit, welches ZD6474 oder ein pharmazeutisch annehmbares Salz davon und Bevacizumab enthält.

8. ZD6474 oder ein pharmazeutisch annehmbares Salz davon und Bevacizumab zur Verwendung bei der Behandlung von Krebs nach Anspruch 1 oder 2, wobei ZD6474 oder ein pharmazeutisch annehmbares Salz davon und Bevacizumab gleichzeitig, aufeinanderfolgend oder getrennt verabreicht werden.

9. ZD6474 oder ein pharmazeutisch annehmbares Salz davon und Bevacizumab und ein Inhibitor der Tyrosinkinase des epidermen Wachstumsfaktorrezeptors zur Verwendung bei der Behandlung von Krebs in einem Warmblüter wie einem Menschen.

## Revendications

1. ZD6474 ou un sel pharmaceutiquement acceptable de celui-ci et bévacizumab pour utilisation dans le traitement du cancer chez un animal à sang chaud tel qu'un humain.

2. ZD6474 ou un sel pharmaceutiquement acceptable de celui-ci et bévacizumab pour utilisation dans le traitement du cancer selon la revendication 1 dans lequel l'animal à sang chaud tel qu'un humain est traité avec un rayonnement ionisant.

3. ZD6474 ou un sel pharmaceutiquement acceptable de celui-ci et bévacizumab pour utilisation dans le traitement du cancer selon la revendication 1 ou la revendication 2, le cancer étant un cancer colorectal, un cancer du poumon, comprenant un mésothéliome pleural malin, un cancer du poumon à petites cellules (SCLC) et un cancer du poumon non à petites cellules (NSCLC), un cancer du sein, un cancer rénal, un cancer de la vessie, un cancer de l'oesophage, un cancer pancréatique, un cancer de la tête et du cou, un cancer de la thyroïde, un cancer de la peau comprenant un mélanome, un carcinome hépatocellulaire, un gliome, un neuroblastome, un cancer gastrique, un cancer de la prostate, un cancer cervical, un cancer de la vulve ou un cancer ovarien ou une des malignités hématologiques.

4. ZD6474 ou un sel pharmaceutiquement acceptable de celui-ci et bévacizumab pour utilisation dans le traitement du cancer selon la revendication 1 ou la revendication 2, le cancer étant un cancer du poumon non à petites cellules (NSCLC) ou un cancer colorectal ou un cancer du rein, du cerveau ou du sein.

5. ZD6474 ou un sel pharmaceutiquement acceptable de celui-ci et bévacizumab pour utilisation dans le traitement du cancer selon la revendication 1 ou la revendication 2, le cancer étant cancer du poumon non à petites cellules (NSCLC) ou un cancer colorectal.

6. Composition pharmaceutique qui comprend ZD6474 ou un sel pharmaceutiquement acceptable de celui-ci, et bévacizumab en association avec un excipient ou véhicule pharmaceutiquement acceptable.

7. Kit comprenant ZD6474 ou un sel pharmaceutiquement acceptable de celui-ci e t bévacizumab.

8. ZD6474 ou un sel pharmaceutiquement acceptable de celui-ci et bévacizumab pour utilisation dans le traitement du cancer selon la revendication 1 ou la revendication 2, ZD6474 ou un sel pharmaceutiquement acceptable de celui-ci et le bévacizumab étant administrés simultanément, séquentiellement ou séparément.

9. ZD6474 ou un sel pharmaceutiquement acceptable de celui-ci et bévacizumab et un inhibiteur de récepteur tyrosine kinase de facteur de croissance épidermique pour utilisation dans le traitement du cancer chez un animal à sang chaud tel qu'un humain.
